# EUROPEAN PATENT APPLICATION

(11) **EP 2 098 525 A1**
(43) Date of publication of application: **09.09.2009**
(21) Application number: 08101985.3
(22) Date of filing: 26.02.2008
(51) Int. Cl.: C07D 477/20, A61K 31/4025, A61P 31/04

(54) **Crystalline carbapenem compound and produced method thereof**

(71) Applicant: Savior Lifetec Corporation, 350 MiaLi County (TW)
(72) Inventor: Tseng, Wei-Hong, MiaLi County 350 (TW); Su, Yu-Lun, MiaLi County 350 (TW); Yu, Chiung-Yi, MiaLi County 350 (TW)
(74) Representative: Lermer, Christoph

(57) **Abstract**

The present invention relates to a crystalline carbapenem compound and produced method thereof. The crystalline carbapenem compound of the present invention is crystalline (4*R*,5*S*,6*S*)-3-[[(3*S*,5*S*)-5-[(dimethylamino)carbonyl]-3-pyrrolidinyl]thio]-6-[(1*R*)-1-hydroxyethyl]-4-methyl-7-oxo-1-azabicyclo[3.2.0] hept-2-ene-2-carboxylic acid and it is characterized by an X-ray powder diffraction pattern of Fig. 2. The crystalline carbapenem compound of the present invention is a new crystalline form and is useful as an antibiotic agent. Furthermore, the crystalline carbapenem compound of the present invention is much more stable than carbapenem compound in a non-crystalline form. Hence, the crystalline carbapenem compound of the present invention is suitable for storage.

## Description

### Background of the Invention

### Field of the Invention

The present invention relates to a carbapenem compound and produced method thereof, and more particular, to a crystalline carbapenem compound and produced method thereof, which is used for the antibiotic agent and is much more stable and suitable for storage.

### Description of the Related Art

The carbapenem compound is (4*R*,5*S*,6*S*)-3-[[(3*S*,5*S*)-5-[(dimethylamino)carbonyl]-3-pyrrolidinyl]thio]-6-[(1*R*)-1-hydroxyethyl]-4-met hyl-7-oxo-1-azabicyclo[3.2.0]hept-2-ene-2-carboxylic acid (hereinafter referred to as "Compound A") of the formula: 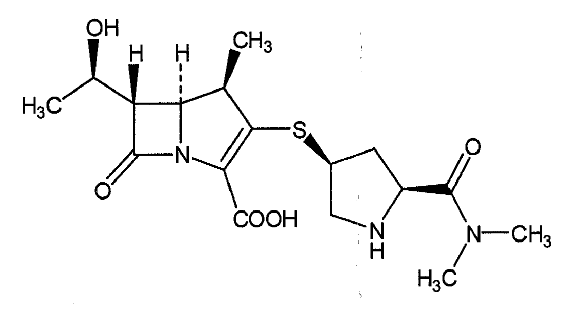 , which is useful as an antibiotic agent.

Compound A in a non-crystalline form, i.e. as an amorphous powder obtained by lyophilization, and its use as an antibiotic agent are reported in EP 126587A. However, Compound A in such non-crystalline form is not sufficiently stable and decomposes with decrease of its antibiotic potency during the storage over a long period of time.

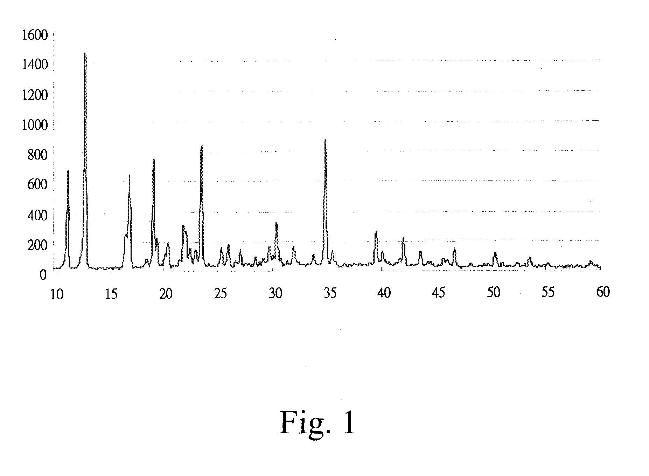

In EP 256377 and USP 4888344 there been obtained Compound A in a crystalline form A with a high purity. Further, it has been found that Compound A in such crystalline form A is much more stable than in a non-crystalline form and suitable for storage. The crystalline form A is characterized by an X-ray powder diffraction pattern of Fig. 1. Furthermore, the X-ray powder diffraction pattern of Fig. 1 includes the spacing in lattice and the relative intensity value as set forth in Table 1.

**Table 1 X-ray powder diffraction pattern of Fig. 1 including the spacing in lattice and the relative intensity value**

| (Spacing in lattice) | I/I*i* (Relative intensity) |
|---|---|
| 1.81 | 5 |
| 1.95 | 4 |
| 2.04 | 6 |
| 2.15 | 7 |
| 2.45 | 8 |
| 2.29 | 12 |
| 2.37 | 4 |
| 2.39 | 4 |
| 2.53 | 10 |
| 2.58 | 17 |
| 2.66 | 7 |
| 2.80 | 15 |
| 2.86 | 7 |
| 2.94 | 16 |
| 3.01 | 9 |
| 3.07 | 9 |
| 3.14 | 9 |
| 3.30 | 16 |
| 3.35 | 6 |
| 3.44 | 8 |
| 3.52 | 16 |
| 3.79 | 31 |
| 3.88 | 14 |
| 3.96 | 20 |
| 4.04 | 28 |
| 4.34 | 12 |
| 4.41 | 10 |
| 4.57 | 12 |
| 4.64 | 34 |
| 4.80 | 15 |
| 5.15 | 2 |
| 5.25 | 71 |
| 5.35 | 39 |
| 6.71 | 2 |
| 6.89 | 100 |
| 7.88 | 30 |

As the result of the extensive study, the present invention has now been succeeded in obtained Compound A in a new crystalline form B with a high purity and stable for storage.

### SUMMARY OF THE INVENTION

A primary objective of the present invention is to provide a crystalline carbapenem compound and produced method thereof. The crystalline carbapenem compound is a new crystalline form.

Another objective of the present invention is to provide a crystalline carbapenem compound and produced method thereof. The crystalline carbapenem compound is much more stable than carbapenem compound in a non-crystalline form.

Next objective of the present invention is to provide a crystalline carbapenem compound and produced method thereof. The crystalline carbapenem compound is suitable for storage.

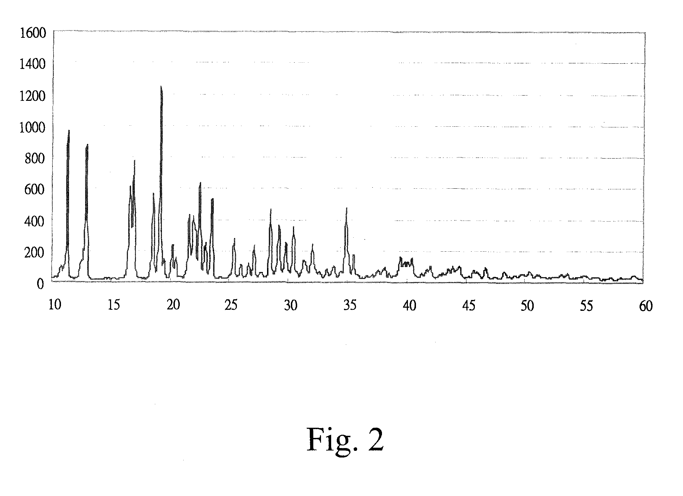

A crystalline carbapenem compound of the present invention comprises:
crystalline (4*R*,5*S*,6*S*)-3-[[(3*S*,5*S*)-5-[(dimethylamino)carbonyl] -3-pyrrolidinyl]thio]-6-[(1*R*)-1-hydroxyethyl]-4-methyl-7-oxo-1-azabicyclo[3.2 .0]hept-2-ene-2-carboxylic acid of the formula: 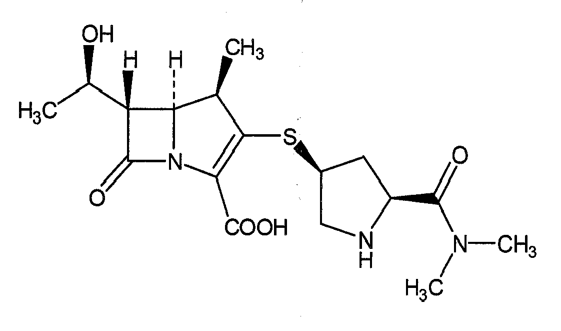
Wherein the crystalline (4*R*,5*S*,6*S*)-3-[[(3*S*,5*S*)-5-[(dimethylamino) carbonyl]-3-pyrrolidinyl]thio]-6-[(1*R*)-1-hydroxyethyl]-4-methyl-7-oxo-1-azab icyclo[3.2.0]hept-2-ene-2-carboxylic acid (Compound A in a new crystalline form B) is characterized by an X-ray powder diffraction pattern of Fig. 2 of the specification.

A produced method of crystalline carbapenem compound of the present invention comprises the steps of: (1) activated carbon being added to solution and stirring, the solution containing crude product of 4*R*,5*S*,6*S*)-3-[[(3*S*,5*S*)-5-[(dimethylamino)carbonyl] -3-pyrrolidinyl]thio]-6-[(1*R*)-1-hydroxyethyl]-4-methyl-7-oxo-1-azabicyclo[3.2 .0]hept-2-ene-2-carboxylic acid and water; (2) the activated carbon being removed from the solution by a filtration and filtrate being washed with water to form aqueous solution; (3) the aqueous solution being concentrated by a reverse osmosis condensing apparatus and resulting condensate being cooled; (4) a seed being added to the resulting condensate; (5) solvent being added thereto and then stirring; and (6) precipitated crystals being collected, washed and dried to get crystalline (4*R*,5*S*,6*S*)-3-[[(3*S*,5*S*)-5-[(dimethylamino) carbonyl]-3-pyrrolidinyl]thio]-6-[(1*R*)-1-hydroxyethyl]-4-methyl -7-oxo-1-azabicyclo[3.2.0]hept-2-ene-2-carboxylic acid (compound A in a new crystalline form B), which is characterized by an X-ray powder diffraction pattern of Fig. 2.

### Brief Description of the Drawings

- Fig. 1: is X-ray powder diffraction pattern of carbapenem compound in crystalline form A of the prior art.
- Fig. 2: is X-ray powder diffraction pattern of crystalline carbapenem compound of the present invention.
- Fig. 3: is a flowchart of the produced method of crystalline carbapenem compound of the present invention.

### Detailed Description of the Preferred Embodiment

A crystalline carbapenem compound of present invention comprises crystalline (4*R*,5*S*,6*S*)-3-[[(3*S*,5*S*)-5-[(dimethylamino)carbonyl]-3-pyrrolidinyl]thio]-6-[(1*R*)-1-hydroxyethyl]-4-methyl-7-oxo-l-azabicyclo [3.2.0]hept-2-ene-2-carboxylic acid, whose formula is shown as following:

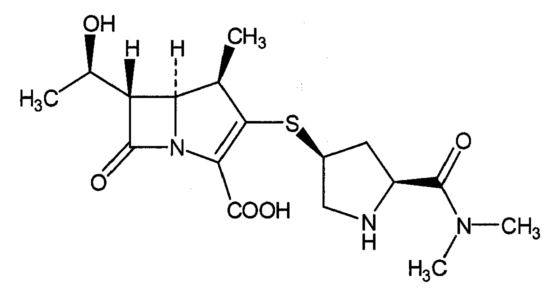

The crystalline (4*R*,5*S*,6*S*)-3-[[(3*S*,5*S*)-5-[(dimethylamino)carbonyl] -3-pyrrolidinyl]thio]-6-[(1*R*)-1-hydroxyethyl]-4-methyl-7-oxo-1-azabicyclo[3-2 .0]hept-2-ene-2-carboxylic acid (compound A in a new crystalline form B) is characterized by an X-ray powder diffraction pattern of Fig. 2. Furthermore, the X-ray powder diffraction pattern of Fig. 2 includes the spacing in lattice and the relative intensity value as set forth in Table 2.

**Table 2 X-ray powder diffraction pattern of Fig. 2 including the spacing in lattice and the relative intensity value**

| (Spacing in lattice) | I/I*i* (Relative intensity) |
|---|---|
| 1.81 | 8.0 |
| 1.94 | 10.8 |
| 2.07 | 9.1 |
| 2.14 | 15.4 |
| 2.24 | 9.0 |
| 2.27 | 18.1 |
| 2.52 | 9.5 |
| 2.57 | 60.3 |
| 2.65 | 7.6 |
| 2.79 | 11.3 |
| 2.93 | 22.2 |
| 3.00 | 11.3 |
| 3.13 | 6.6 |
| 3.28 | 9.7 |
| 3.42 | 12.3 |
| 3.50 | 11.2 |
| 3.79 | 57.3 |
| 3.86 | 10.1 |
| 3.95 | 10.7 |
| 4.05 | 21.3 |
| 4.33 | 12.8 |
| 4.39 | 8.0 |
| 4.53 | 10.7 |
| 4.62 | 51.0 |
| 5.22 | 44.2 |
| 5.36 | 15.0 |
| 6.85 | 100.0 |
| 7.81 | 46.6 |

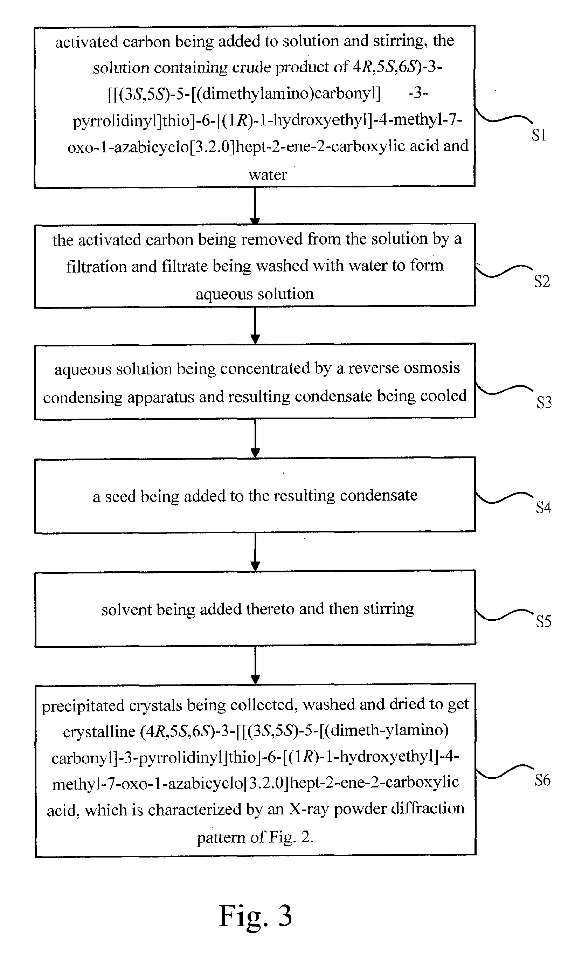

A produced method of crystalline carbapenem compound of present invention comprises the steps of (as shown in Fig. 3):
(1) activated carbon being added to solution and stirring, the solution containing crude product of 4*R*,5*S*,6*S*)-3-[[(3*S*,5*S*)-5-[(dimethylamino)carbonyl] -3-pyrrolidinyl]thio]-6-[(1*R*)-1-hydroxyethyl]-4-methyl-7-oxo-1-azabicyclo [3.2.0]hept-2-ene-2-carboxylic acid and water;
(2) the activated carbon being removed from the solution by a filtration and filtrate being washed with water to form aqueous solution;
(3) aqueous solution being concentrated by a reverse osmosis condensing apparatus and resulting condensate being cooled;
(4) a seed being added to the resulting condensate;
(5) solvent being added thereto and then stirring; and
(6) precipitated crystals being collected, washed and dried to get crystalline (4*R*,5*S*,6*S*)-3-[[(3*S*,5*S*)-5-[(dimethylamino)carbonyl]-3-pyrrolidinyl]thio] -6-[(1*R*)-1-hydroxyethyl]-4-methyl-7-oxo-1-azabicyclo[3.2.0]hept-2-ene-2-carboxylic acid, which is characterized by an X-ray powder diffraction pattern of Fig. 2.

The stirring time of the step (1) is 1∼3 hours. In the step (1), the weight ratio of the crude product of (4*R*,5*S*,6*S*)-3-[[(3*S*,5*S*)-5-[(dimethylamino)carbonyl] -3-pyrrolidinyl]thio] -6-[(1*R*)-1-hydroxyethyl]-4-methyl-7-oxo-1-azabicyclo[3.2.0]hept-2-ene -2-carboxylic acid and the activated carbon is 100 : 1-30.

The aqueous solution being concentrated by the reverse osmosis condensing apparatus of the step (3) is under about 50∼300 psi at 5-20□. The resulting condensate is cooled to 0-15□ in the step (3). The seed comprises ≧ 95 wt% Meropenem. The solvent of the step (5) is selected from the consisting of methanol, ethanol, tetrahydrofuran, acetone, isopropyl alcohol and 1-propanol, methyl acetate, ethyl acetate, methyl ethyl ketone, methyl isobutyl ketone and mixture(s) thereof. The addition step of the step (5) is at -5 to 20□. The stirring step of the step (5) is more than 3 hours at 5 to -30□.
The precipitated crystals are collected by filtration and the precipitated crystals are washed by methanol, ethanol, tetrahydrofuran, acetone, isopropyl alcohol and 1-propanol, methyl acetate, ethyl acetate, methyl ethyl ketone, methyl isobutyl ketone and mixturing thereof. in the step (6). Also, the precipitated crystals are dried at 10-35□ for 3-6 hours in the step (6).

### Example 1

Crude product of (4*R*,5*S*,6*S*)-3-[[(3*S*,5*S*)-*5-*[(dimethylamino)carbonyl]-3-pyrrolidinyl]thio]-6-[(1*R*)-1-hydroxyethyl]-4-met hyl-7-oxo-1-azabicyclo[3.2.0]hept-2-ene-2-carboxylic acid (content, 95%; 200g) was dissolved in water (about 20L) at 20□, activated carbon (10g) was added thereto, and stirred for about one hour. After removal of the activated carbon by filtration, the filtrate was washed with water; the aqueous solution was concentrated by the use of reverse osmosis condensing apparatus under a pressure of about 120 psi at 10-20□. The resulting condensate (6.7L) was cooled to 0 to 10□. The seed (Meropenem with 98% content; 1g) was added to the resulting condensate, and then a (20.1L) tetrahydrofuran was added thereto at 0-10□, followed by stirring for 6 hour at 0-5□. The precipitated crystals were collected by filtration, washed with acetone and dried at 30□ for 3 hours to give 180.5g of the crystalline carbapenem compound (compound A in a crystalline form B) of the present invention.

### Example 2

Crude product of (4*R*,5*S*,6*S*)-3-[[(3*S*,5*S*)-5-[(dimethylamino)carbonyl]-3-pyrrolidinyl]thio]-6-[(1*R*)-1-hydroxyethyl]-4-met hyl-7-oxo-1-azabicyclo[3.2.0]hept-2-ene-2-carboxylic acid (content, 95%; 200g) was dissolved in water (about 20L), activated carbon (10g) was added thereto, and stirred for about one hour. After removal of the activated carbon by filtration, the filtrate was washed with water; the aqueous solution was concentrated by the use of reverse osmosis condensing apparatus under a pressure of about 200 psi at 5-15□. The resulting condensate (6.7L) was cooled to 0 to 10□. The seed (Meropenem with 98% content; 1g) was added to the resulting condensate, and then acetone (20.1L) was added thereto, followed by stirring for 8 hour at -5 to -15□. The precipitated crystals were collected by filtration, washed with tetrahydrofuran and dried at 15-25□ for 2 hours to give 175.5g of the crystalline carbapenem compound of the present invention.

### Example 3

Crude product of (4*R*,5*S*,6*S*)-3-[[(3*S*,5*S*)-5-[(dimethylamino)carbonyl]-3-pyrrolidinyl]thio]-6-[(1*R*)-1-hydroxyethyl]-4-met hyl-7-oxo-1-azabicyclo[3.2.0]hept-2-ene-2-carboxylic acid (content, 95%; 200g) was dissolved in water (about 20L), activated carbon (10g) was added thereto, and stirred for about one hour. After removal of the activated carbon by filtration, the filtrate was washed with water; the aqueous solution was concentrated by the use of reverse osmosis condensing apparatus under a pressure of about 150 psi at 15-20□. The resulting condensate (6.7L) was cooled to about 100. The seed (Meropenem with 98% content; 1g) was added to the resulting condensate, and then isopropyl alcohol (20.1L) was added thereto, followed by stirring for 4 hour at -5 to -150. The precipitated crystals were collected by filtration, washed with tetrahydrofuran and dried at 25-30□ for 5 hours to give 170.5g of the crystalline carbapenem compound of the present invention.

### Example 4

Crude product of (4*R*,5*S*,6*S*)-3-[[(3*S*,5*S*)-5-[(dimethylamino)carbonyl]-3-pyrrolidinyl]thio]-6-[(1*R*)-1-hydroxyethyl]-4-met hyl-7-oxo-1-azabicyclo[3.2.0]hept-2-ene-2-carboxylic acid (content, 95%; 200g) was dissolved in water (about 20L), activated carbon (10g) was added thereto, and stirred for about one hour. After removal of the activated carbon by filtration, the filtrate was washed with water; the aqueous solution was concentrated by the use of reverse osmosis condensing apparatus under a pressure of about 50 psi at 15-20□. The resulting condensate (6.7L) was cooled to about 15□. The seed (Meropenem with 98% content; 1g) was added to the resulting condensate, and then 1-propanol (20.1L) was added thereto, followed by stirring for 4 hour at -10-20□. The precipitated crystals were collected by filtration, washed with acetone and dried at 20 to 35□ for 6 hours to give 168.5g of the crystalline carbapenem compound of the present invention.

Accordingly, the present invention has following features:
1. The crystalline carbapenem compound of the present invention is a new crystalline form and used for antibiotic agent.
2. The crystalline carbapenem compound of the present invention is much more stable than carbapenem compound in a non-crystalline form.
3. The crystalline carbapenem compound of the present invention is suitable for storage.

While certain preferred embodiments of the present invention have been disclosed in detail, it is to be understood that various modifications may be adopted without departing from the spirit of the invention or scope of the following claims.

## Claims

1. A crystalline carbapenem compound comprising:
crystalline (4*R*,5*S*,6*S*)-3-[[(3*S*,5*S*)-5-[(dimethylamino)carbonyl] -3-pyrrolidinyl]thio]-6-[(1*R*)-1-hydroxyethyl]-4-methyl-7-oxo-1-azabicycl 0[3.2.0]hept-2-ene-2-carboxylic acid of the formula: 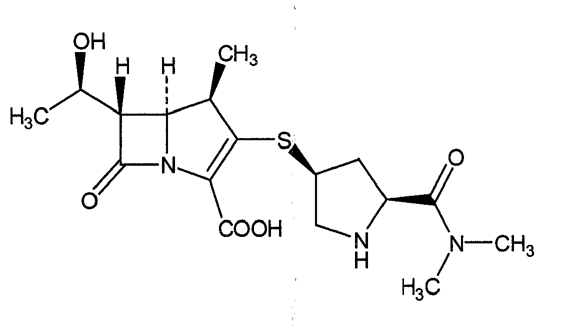
Wherein the crystalline (4*R*,5*S*,6*S*)-3-[[(3*S*,5*S*)-5-[(dimethylamino) carbonyl]-3-pyrrolidinyl]thio]-6-[(1*R*)-1-hydroxyethyl]-4-methyl-7-oxo-1 -azabicyclo[3.2.0]hept-2-ene-2-carboxylic acid is **characterized by** an X-ray powder diffraction pattern of Fig. 2 of the specification.

2. The crystalline carbapenem compound according to claim 1, the crystalline (4*R*,5*S*,6*S*)-3-[[(3*S*,5*S*)-5-[(dimethylamino)carbonyl]-3-pyrrolidinyl] thio]-6-[(1*R*)-1-hydroxyethyl]-4-methyl-7-oxo-1-azabicyclo[3.2.0]hept-2-ene-2-carboxylic acid is **characterized by** an X- ray powder diffraction pattern including spacing in lattice and relative intensity value as set forth in Table 2 of the specification.

3. A produced method of crystalline carbapenem compound comprising the steps of:
(1) activated carbon being added to solution and stirring, the solution containing crude product of 4*R*,5*S*,6*S*)-3-[[(3*S*,5*S*)-5-[(dimethylamino)carbonyl] -3-pyrrolidinyl]thio]-6-[(1*R*)-1-hydroxyethyl]-4-methyl-7-oxo-1-azabicyclo [3.2.0]hept-2-ene-2-carboxylic acid and water;
(2) the activated carbon being removed from the solution by a filtration and filtrate being washed with water to form aqueous solution;
(3) the aqueous solution being concentrated by a reverse osmosis condensing apparatus and resulting condensate being cooled;
(4) a seed being added to the resulting condensate;
(5) solvent being added thereto and then stirring; and
(6) precipitated crystals being collected, washed and dried to get crystalline (4*R*,5*S*,6*S*)-3-[[(3*S*,5*S*)-5-[(dimethylamino)carbonyl]-3-pyrrolidinyl]thio]-6-[(1*R*)-1-hydroxyethyl]-4-methyl-7-oxo-1-azabicyclo[3.2 .0]hept-2-ene-2-carboxylic acid, which is **characterized by** an X-ray powder diffraction pattern of Fig. 2 of the specification.

4. The produced method of crystalline carbapenem compound according to claim 3, wherein the weight ratio of the crude product of (4*R*,5*S*,6*S*)-3-[[(3*S*,5*S*)-5-[(dimethylamino)carbonyl] -3-pyrrolidinyl]thio] -6-[(1*R*)-1-hydroxyethyl]-4-methyl-7-oxo-1-azabicyclo[3.2.0]hept-2-ene -2-carboxylic acid and the activated carbon is 100 : 1-30.

5. The produced method of crystalline carbapenem compound according to claim 3, wherein the aqueous solution being concentrated by the reverse osmosis condensing apparatus of the step (3) is under about 50∼300 psi at 5-20□.

6. The produced method of crystalline carbapenem compound according to claim 3, wherein the seed comprises ≧95 wt% Meropenem in the step (4).

7. The produced method of crystalline carbapenem compound according to claim 3, wherein the solvent of the step (5) is selected from the consisting of methanol, ethanol, tetrahydrofuran, acetone, isopropyl alcohol, 1-propanol, methyl acetate, ethyl acetate, methyl ethyl ketone, methyl isobutyl ketone and mixturing thereof.

8. The produced method of crystalline carbapenem compound according to claim 3, wherein the addition of solvent of the step (5) is at -5 to 20 □.

9. The produced method of crystalline carbapenem compound according to claim 3, wherein the stirring step is cooled to 10 to -30□.

10. The produced method of crystalline carbapenem compound according to claim 3 in the step (6), wherein the precipitated crystals are washed by methanol, ethanol, tetrahydrofuran, acetone, isopropyl alcohol, 1-propanol, methyl acetate, ethyl acetate, methyl ethyl ketone, methyl isobutyl ketone and mixturing thereof.

11. The produced method of crystalline carbapenem compound according to claim 3, wherein the precipitated crystals are dried at 10 to 35□ in the step (6).

12. The produced method of white crystalline carbapenem compound according to claim 3, wherein the crystalline (4*R*,5*S*,6*S*)-3-[[(3*S*,5*S*)-5-[(dimethylamino)carbonyl]-3-pyrrolidinyl]thio]-6 -[(1*R*)-1-hydroxyethyl] -4-methyl -7-oxo-1-azabicyclo[3.2.0] hept-2-ene-2-carboxylic acid of the step (6) is **characterized by** an X- ray powder diffraction pattern including spacing in lattice and relative intensity value as set forth in Table 2 of the specification.
